# EUROPEAN PATENT APPLICATION

(11) **EP 4 180 406 A1**
(43) Date of publication of application: **17.05.2023**
(21) Application number: 22208764.5
(22) Date of filing: 28.07.2017
(51) Int. Cl.: C04B 35/486, A61K 6/00, C04B 41/85, C04B 41/00, A61C 13/08, C04B 41/45, C04B 41/52, C04B 41/89, A61K 6/16, A61K 6/78, A61K 6/802, A61K 6/818, A61C 13/00

(54) **DENTAL TEMPORARY CALCINED BODY HAVING COLORED INSIDE**

(30) Priority: 29.07.2016 JP 2016149277
(62) Divisional of application: 17183791.7
(71) Applicant: Shofu Inc., Kyoto 605-0983 (JP)
(72) Inventor: KADOBAYASHI, Yusei, KYOTO, 605-0983 (JP); SHIGEZAWA, Masako, KYOTO, 605-0983 (JP)
(74) Representative: Eisenführ Speiser

(57) **Abstract**

To provide a dental temporary calcined body including an inside colored by a coloring material wherein, the inside of the dental temporary calcined body is colored by a coloring liquid containing the coloring material and a penetrant containing a permeating liquid.

## Description

### CROSS-REFERENCE TO RELATED APPLICATIONS

This application is based on and claims the benefit of priority from Japanese Patent Application Serial No. 2016-149277 (filed on Jul. 29 2016), the contents of which are hereby incorporated by reference in their entirety.

### BACKGROUND OF THE INVENTION

### Field of the Invention

The present disclosure relates to a colored dental temporary calcined body for preparing a dental restoration by using CAD/CAM.

### Description of the Related Art

A conventional dental restoration has been prepared by cutting a dental temporary calcined body by using CAD/CAM technique. For example, a dental restoration has been prepared by cutting a dental temporary calcined block into a form of a dental restoration having a form of a part of the natural tooth form and calcining the cut dental temporary calcined block.

The prepared dental restoration is required to be colored with a natural tooth color. Therefore, various coloring techniques for a dental temporary calcined block have been developed.

PCT International Publication No. WO 2009/154301 discloses a dental temporary calcined body prepared by stacking powder materials which are colored with a plurality of colors, in order to obtain a dental restoration colored with a natural tooth color. However, because a plurality of powder materials are used, a peeling of an interface may occur. Further, it is impossible to reproducing a complex coloring.

Japanese Unexamined Patent Application Publication (Translation of PCT Application) No. 2002-536280 (JP 2002-536280 A) and Japanese Unexamined Patent Application Publication (Translation of PCT Application) No. JP2010-534245 (JP 2010-534245 A) disclose a colored translucency dental restoration prepared by immersing a dental restoration into a metal ion coloring liquid or a metal complex coloring liquid and calcining the immersed dental restoration. However, because coloring of single color is done on only the surface, it is impossible to reproduce a coloration in an inside of a dental restoration.

### SUMMARY OF THE INVENTION

### Technical Problem

It has been required to reproduce a natural tooth color in one dental temporary calcined body by using a plurality of colors. Further, a technique for reproducing a complex color has been also required.

### Solution to Problem

The present disclosure provides a dental temporary calcined body including an inside colored by a coloring material wherein, the inside of the dental temporary calcined body is colored by a coloring liquid containing the coloring material and a penetrant containing a permeating liquid.

The present disclosure also provides a dental temporary calcined body including an inside colored by a coloring material wherein, the inside of the dental temporary calcined body is permeated with the coloring material by infiltrating a first coloring liquid containing a first coloring material into the dental temporary calcined body, and by infiltrating a second coloring liquid containing a second coloring material having a color different from the color of the first coloring material or a penetrant containing a permeating liquid into the dental temporary calcined body.

The present disclosure further provides a method for preparing a dental temporary calcined body including an inside colored by a coloring material, wherein the method includes infiltrating a first coloring liquid containing a first coloring material into a temporary calcined body, and infiltrating a second coloring liquid containing a second coloring material having a color different from the color of the first coloring material or a penetrant containing a permeating liquid into the temporary calcined body to infiltrating the coloring material into the inside of the temporary calcined body.

In addition, the present disclosure provides a dental calcined body obtained by cutting and machining a dental temporary calcined body prepared by the method of the present disclosure into a form of a dental restoration and calcining the cut dental temporary calcined body.

### Advantageous Effects of Invention

According to the present disclosure, a complex coloration of a tooth substance may be reproduced in an inside of a dental temporary calcined body and the inside of a dental temporary calcined body may be colored freely and three-dimensionally. As a result, a dental restoration obtained from a dental temporary calcined body according to the present disclosure has excellent esthetic property, high color reproducibility, and an excellent designability.

In the conventional dental temporary calcined body, it is necessary that a plurality of dental temporary calcined bodies according to colors of shade guides are held. In contrast, in the present disclosure, because a coloration is applied on a temporary calcined body, it may be sufficient to hold one kind of uncolored temporary calcined body, a coloring liquid containing a coloring material and a penetrant containing a permeating liquid.

In the present disclosure, because an uncolored temporary calcined body is colored individually, a coloration may be applied according to patient's condition and requests of dentists and dental technicians. Further, in the present disclosure, because a temporary calcined body does not have a plurality of divided layers respectively including a powder of colored ceramics, an obtained dental temporary calcined body and an obtained dental calcined body have high properties and high aesthetic properties.

According to the present disclosure, because intensity of a coloration may be reproduced into a dental temporary calcined body, shading may be reproduced appropriately. Therefore, a transition portion of colors becomes unclear and aesthetic properties more increase.

### DETAILED DESCRIPTION OF THE PREFERRED EMBODIMENTS

In a temporary calcined body used for a dental temporary calcined body, ceramics are temporary bonded each other by temporary calcination, and there are continuous holes between ceramics particles. These holes may be permeated with a coloring liquid and a penetrant described below. A temporary calcined body may be prepared by placing ceramics into a mold, compression molding the ceramics and temporary calcining the ceramics.

A dental temporary calcined body may be prepared by permeation of (or by infiltrating) a coloring material into a temporary calcined body. It is difficult to direct infiltrate a coloring material into a temporary calcined body. Therefore, a coloring liquid is prepared by mixing a coloring material and a liquid material for coloring, and a dental temporary calcined body is prepared by applying a coloration on a temporary calcined body by permeation of the prepared coloring liquid into the temporary calcined body. A liquid material for coloring is removed after permeation of a coloring material by vaporization or calcination.

A dental restoration having a final form may be obtained by cutting and machining a dental temporary calcined body into a form of the dental restoration which has a form of a part of a natural tooth form, and main calcining the cut dental temporary calcined body. Specifically, when a dental restoration which has a form of a part of a natural tooth form is cut from a dental temporary calcined body, a dental restoration having a final form may be obtained, for example, by cutting and machining a dental temporary calcined body into a large similar form of the dental restoration having the final form by taking into account a shrinkage in a main calcination, and by main calcining the cut dental temporary calcined body. Further, a dental restoration having a final form may be also obtained by cutting and machining a dental calcined body prepared by main calcining a dental temporary calcined body into a form of a dental restoration which has a form of a part of a natural tooth form and has the final form.

A temporary calcination temperature and a main calcination temperature differs depending on a composition and a particle size of ceramics. Generally, a main calcination temperature is higher than a temporary calcination temperature by 100 to 600°C. Preferable temporary calcination temperature is within a range of 800 to 1500 °C, and preferable main calcination temperature is within a range of 1100 to 2100 °C.

A dental temporary calcined body permeated with a coloring liquid containing a coloring material may be temporary calcined one more time. By additional temporary calcination, a liquid material for coloring may be removed and a coloring material may be fixed into a dental temporary calcined body.

A dental temporary calcined body is prepared by infiltrating a coloring liquid containing a coloring material into a temporary calcined body. In this case, a dental temporary calcined body may be prepared from a temporary calcined body having a shape similar to that of the dental temporary calcined body. It is preferable that a temporary calcined body is prepared in largish, and is permeated with a coloring liquid containing a coloring material. Thereafter, a dental temporary calcined body is cut out from the temporary calcined body. The reason is that a dental temporary calcined body which is uniformly permeated with a coloring liquid containing a coloring material and a penetrant containing a permeating liquid, may be easily prepared by this preparing method.

A dental temporary calcined body may have a block shape having a dimension of 0.5 to 3.0 cm (one side). Ceramics is not limited particularly, as long as it is a metal oxide and becomes translucent after main calcination. Specifically, ceramics may include silica, alumina and/or zirconia as a main component in a range of 70 to 100%. An average particle diameter of ceramics may be 1 to 50 µm.

A coloring material is not limited particularly as long as it colors ceramics and the color by the coloring material remains on a temporary calcined body after permeation and calcination. Specifically, a group 3 to 12 transition metal of the periodic table and a metal salt thereof may be used as a coloring material. In addition, rare earth metal and rare earth metal salt may be used as a coloring material. Further, metal salt of oxide, metal salt in the form of oxide, and metal oxide containing anion such as Cl⁻, SO₄²⁻, SO₃²⁻, Br, F , NO₂⁻, and/or NO₃⁻ may be used. It is preferable that a mixture thereof is used, appropriately.

Specific examples of a coloring material include a compound including iron, manganese, copper, chrome, zirconium, cobalt, tin, titanium, nickel, vanadium, erbium, praseodymium, terbium, dysprosium, europium, niobium, neodymium and/or ytterbium.

It is necessary that a coloring material permeates between ceramics in a dental temporary calcined body together with a coloring liquid containing the coloring material. Therefore, a particle diameter of a coloring material is smaller than that of ceramics. It is preferable that a particle diameter of a coloring material is within a range of 1/4 to 1/10000 of a particle diameter of ceramics. When a particle diameter of a coloring material is too large, permeation of ceramics may be prevented. When a particle diameter of a coloring material is too small, coloring may be prevented. Specific particle diameter of a coloring material is within a range of 0.0001 to 10 µm. It is preferable that a coloring material is dissolved in a coloring liquid by ionizing, because a coloring material does not act as physical obstruction for permeation of a coloring liquid and a penetrant into a temporary calcined body.

A liquid material for coloring is used as a carrier medium for infiltrating a coloring material into a temporary calcined body. A liquid material for coloring is not limited particularly, as long as it may be dispersed with a coloring material and may have high wettability to a temporary calcined body. Specifically, a liquid material for coloring includes such as water, methanol, ethanol, n-propanol, isopropyl alcohol, acetone, and/ or methyl ethyl ketone. As such a liquid material for coloring, a mixture thereof may be used. It is preferable that a liquid material for coloring includes water, ethanol, isopropyl alcohol and/ or acetone. It is most preferable that a liquid material for coloring is water.

A coloring liquid is a mixture containing a coloring material and a liquid material for coloring. The compounding ratio of a coloring material in the coloring liquid may be within a range of 0.0001 to 30%, and the compounding ratio of a liquid material for coloring in the coloring liquid is within a range of 70 to 99.9999%. It is preferable that a plurality of coloring liquids which have different colors each other are used. By using a plurality of coloring liquids, a reproduction of a rich color expression may be realized. For densely and stably coloring a temporary calcined body, it is preferable that the coloring liquid permeates the temporary calcined body repeatedly after drying a temporary calcined body permeated with a coloring liquid.

A penetrant includes a permeating liquid and preferably has the same component as that of the liquid material for coloring used in a coloring liquid. A coloring liquid and a penetrant may be permeated by immersion. However, it is preferable that a coloring liquid and a penetrant are permeated by dropping, and dripping amount is within a range of 0.01 to 0.00001 ml.

It is preferable that a permeation of a coloring material into a temporary calcined body may be achieved by using a plurality of coloring liquids and a penetrant. For example, different color portions are freely reproduced in a temporary calcined body by infiltrating a second coloring liquid on a portion where a first coloring liquid having different color from that of the second coloring liquid has been permeated, or by adjacently infiltrating a plurality of coloring liquids having different colors each other, and thereby a rich color expression is realized in a temporary calcined body. Uncolored portion may be reproduced by infiltrating a penetrant into a portion where coloring is not applied. Reproduction of color may be achieved by coloring an inside of a temporary calcined body by using a plurality of coloring liquids which have different colors by containing different coloring materials each other.

Specifically, in order to reproduce a mamelon in a dental temporary calcined body, a coloring liquid is applied in mamelon form and permeates. On the other hand, a penetrant is applied on a portion where a permeation of a coloring liquid is not desired, and permeates to prevent an invasion of a coloring liquid into a portion where a coloring is not desired. A mamelon may be reproduced by using a coloring liquid and a penetrant.

In preferable method of minutely infiltrating a coloring liquid and a penetrant for rich color expression, a coloring liquid and/or a penetrant is filled in a nozzle, drops of the coloring liquid and/or the penetrant are prepared at a tip of the nozzle and are jetted to a temporary calcined body, and the temporary calcined body is colored by permeation the jetted drops.

The ink-jet method may be applied to the preparing method of the present invention. By adopting ink-jet method, three-dimensional coloring like a photograph may be applied.

Now a specific description is given.

Zirconia powder having an average particle diameter of 3.0 µm is temporary calcined to prepare a dental temporary calcined body having a quadratic prism shape of 15mm by 15mm. A coloring liquid 1 is prepared by mixing water and iron oxide having an average particle diameter of 0.1 µm and stirring. The proportion of the iron oxide in the coloring liquid is 0.2 wt. %. Hereinafter, the coloring liquid 1 is referred to as "iron oxide coloring liquid". A coloring liquid 2 is prepared by mixing water and cobalt oxide having an average particle diameter of 0.1µm and stirring. The proportion of the cobalt oxide in the coloring liquid is 0.01 wt. %. Hereinafter, the coloring liquid 2 is referred to as "cobalt oxide coloring liquid". As to penetrant, 100 wt. % of water is used.

The temporary calcined body is colored to a thickness of 1mm with the iron oxide coloring liquid by dropping 0.1 to 0.00001 ml of the iron oxide coloring liquid on the temporary calcined body. The temporary calcined body is further colored to a thickness of 1 mm with the cobalt oxide coloring liquid by dropping 0.1 to 0.00001 ml of the cobalt oxide coloring liquid on the surface applied with the iron oxide coloring liquid of the temporary calcined body. The iron oxide coloring liquid and the cobalt oxide coloring liquid permeate an inside of the temporary calcined body by dropping 0.1 to 0.00001 ml of the penetrant on the surface applied with the iron oxide coloring liquid and the cobalt oxide coloring liquid of the temporary calcined body. The dropping of the penetrant is terminated when the iron oxide coloring liquid permeates to a thickness of 5 mm of the temporary calcined body.

A dental temporary calcined body is prepared by drying the temporary calcined body for 1 to 3 hours at 80 to 220 °C. In drying process, a coloring liquid or a penetrant may be moved by drying. Therefore, it is necessary to perform drying carefully or to perform coloring by dropping by taking into account moving of the coloring liquid or the penetrant by drying. A dental calcined body is obtained by a main calcination of a dental temporary calcined body.

The coloring material is freely moved in a temporary calcined body by adjusting a portion in which the coloring liquid is dropped, by adjusting the amount of coloring liquid and by dropping a penetrant. Therefore, a dental temporary calcined body and a dental calcined body which have an inside designed by a coloring material may be obtained.

Mixing coloring liquids and/or a coloring liquid and a penetrant by contacting each other forms a gradation and exhibits shading effect to reproduce a color of a natural tooth. In order to prevent a gradation in initial stage, it is preferable to drop a penetrant firstly, and to drop a coloring liquid thereafter. Gradation and shading may be completely eliminated by using a material which separates a liquid material for coloring.

Next, a specific description is given with reference to the drawings.

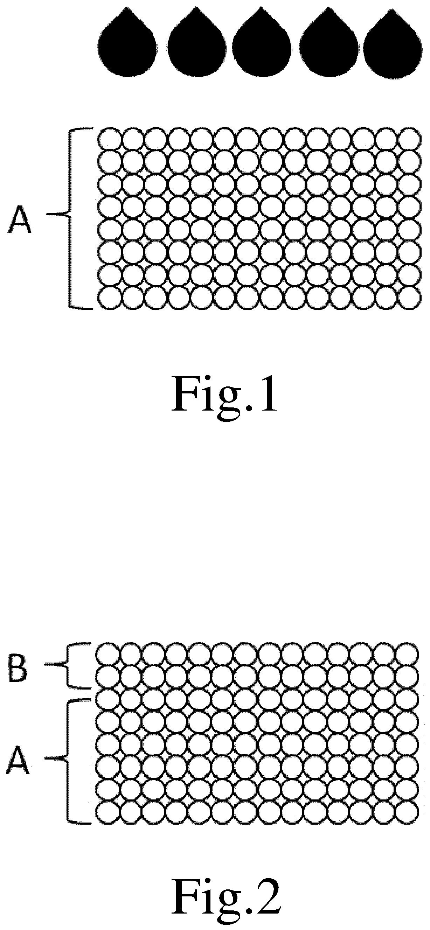

FIG. 1 shows a schematic view showing a state in which an iron oxide coloring liquid is dropped on a temporary calcined body A.

FIG. 2 shows a schematic view showing a state in which the temporary calcined body is permeated with the iron oxide coloring liquid dropped in FIG. 1. In FIG. 2, the portion indicated by "B" is a portion permeated with the iron oxide coloring liquid, and the portion indicated by "A" is a portion permeated with no coloring liquid. In FIG. 2, the number of the layers is two. Further, the thickness of the portion indicated by "B" may be increased by dropping the iron oxide coloring liquid.

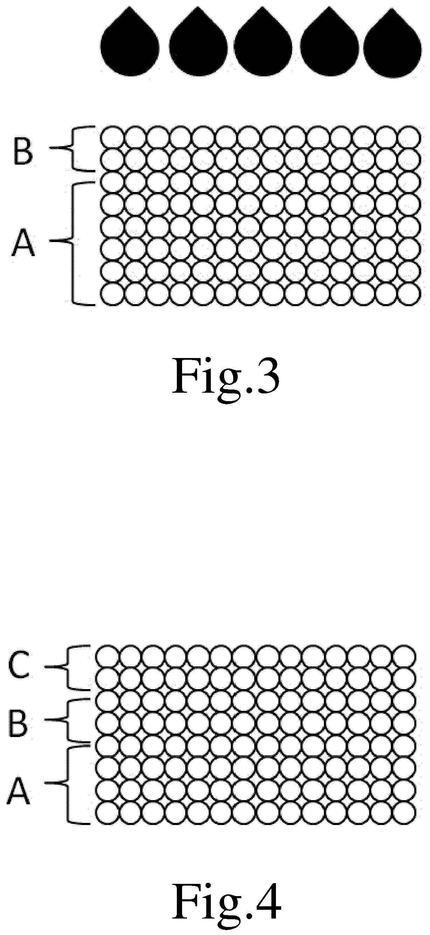

FIG. 3 shows a schematic view showing a state in which a cobalt oxide coloring liquid is dropped on a portion permeated with the iron oxide coloring liquid in the temporary calcined body in FIG. 2.

FIG. 4 shows a schematic view showing a state in which the temporary calcined body is permeated with the cobalt oxide coloring liquid dropped in FIG. 3. The portion indicated by "C" is a portion permeated with the cobalt oxide coloring liquid. The portion indicated by "B" is a portion permeated with the iron oxide coloring liquid. The portion indicated by "A" is a portion permeated with no coloring liquid. In FIG. 4, the number of the layers is three. Further, the thickness of the portion indicated by "C" may be increased by dropping the cobalt oxide coloring liquid. In FIG. 4, a coloring liquid is dropped in the form of a layer. However, a portion colored with both the iron oxide coloring liquid and the cobalt oxide coloring liquid may be obtained by simultaneously dropping the iron oxide coloring liquid and the cobalt oxide coloring liquid.

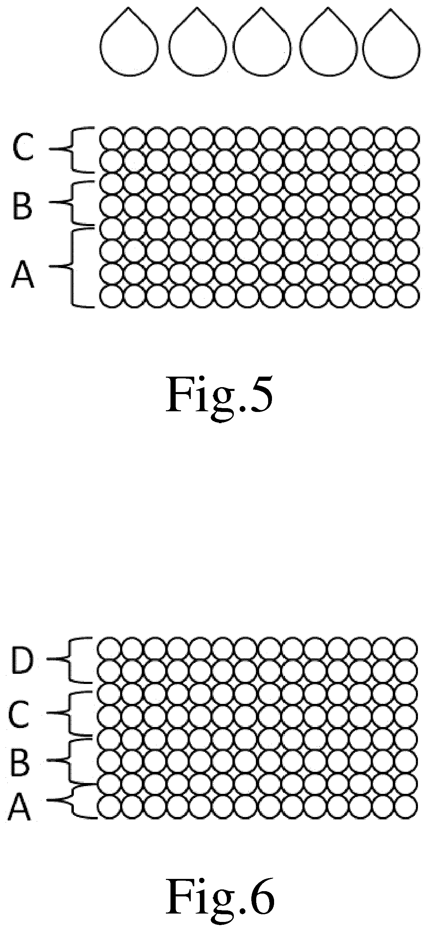

FIG. 5 shows a schematic view showing a state in which a penetrant is further dropped on a portion permeated with the cobalt oxide coloring liquid in the temporary calcined body shown in FIG.4.

FIG. 6 shows a schematic view showing a state in which the temporary calcined body is permeated with the penetrant dropped in FIG.5. The portion indicated by "D" is a portion permeated with the penetrant. The portion indicated by "C" is a portion permeated with the cobalt oxide coloring liquid. The portion indicated by "B" is a portion permeated with the iron oxide coloring liquid. The portion indicated by "A" is a portion permeated with no coloring liquid. In FIG. 6, the number of the layers is four. Further, the thickness of the portion indicated by "D" may be increased by dropping the penetrant. Thus, the colored portion may be further moved by infiltrating various liquids, a coloring material is freely moved to a desired position to color a desired position.

Next, a method of dropping a coloring liquid on a portion of a temporary calcined body is described.

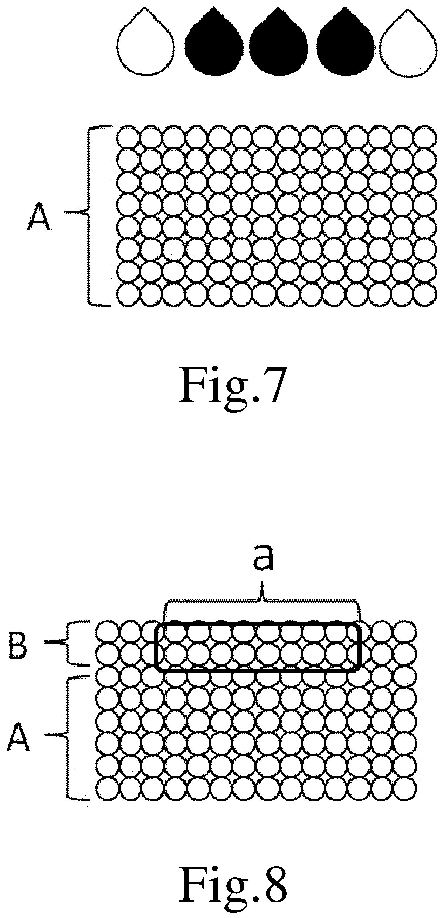

FIG. 7 shows a schematic view showing a state in which a coloring liquid is dropped on a temporary calcined body A. In FIG. 7, three inner dropping liquids are iron oxide coloring liquid and two outer dropping liquids which is arranged on both sides of three inner dropping liquids are penetrants. Because two outer dropping liquids are penetrants which does not color the temporary calcined body, only the portion permeated with the coloring liquids in the temporary calcined body is colored. Other coloring liquid having other color such as cobalt oxide coloring liquid may be used instead of the penetrant. As a result, one layer may be colored with different colors.

FIG. 8 shows a schematic view showing a state in which the temporary calcined body is permeated with the iron oxide coloring liquid dropped in FIG. 7. In FIG. 8, the portion surrounded by the black circle and indicated by both "B" and "a" is a portion permeated with the iron oxide coloring liquid. The portion indicated by "A" is a portion not permeated with the iron oxide coloring liquid. The portion indicated by "B" and not indicated by "a" is a portion permeated with the penetrant. The thickness of the portion indicated by "B" may be increased by dropping the iron oxide coloring liquid and the penetrant.

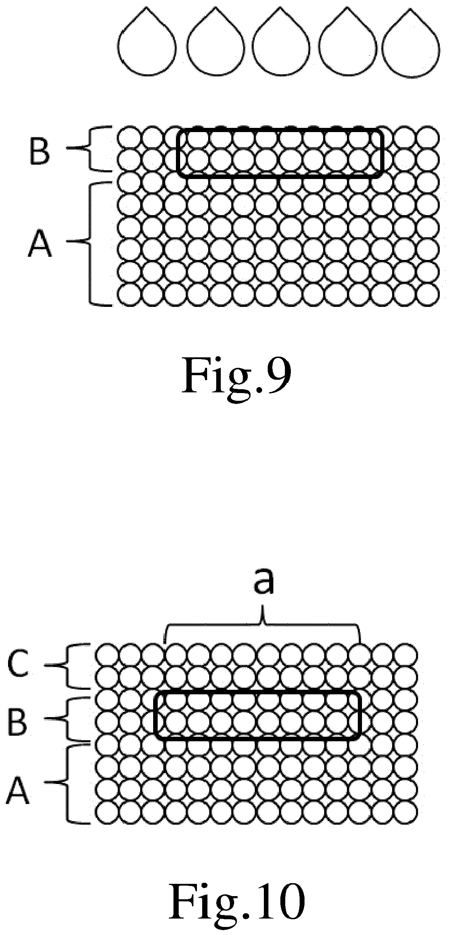

FIG. 9 shows a schematic view showing a state in which a penetrant is further dropped on a portion permeated with the iron oxide coloring liquid and the penetrant in the temporary calcined body shown in FIG. 8.

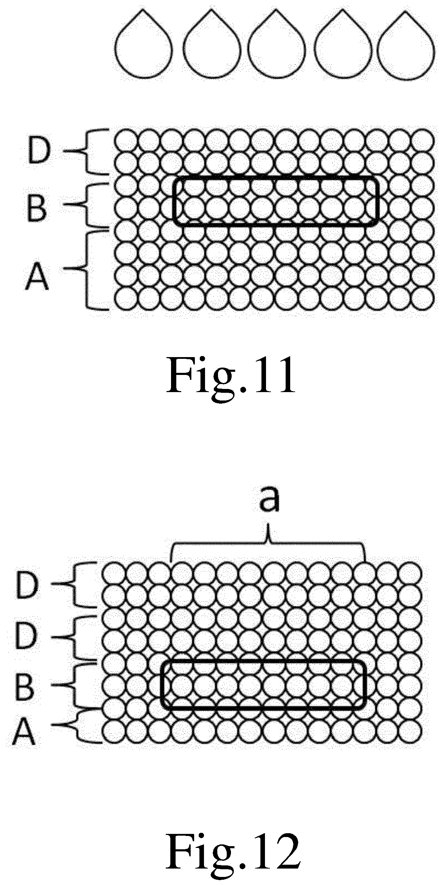

FIG. 10 shows a schematic view showing a state in which the temporary calcined body is permeated with the penetrant dropped in FIG. 9. The portion indicated by "D" is a portion permeated with the penetrant. The portion surrounded by the black circle and indicated by both "B" and "a" is a portion permeated with the iron oxide coloring liquid. The portion indicated by "B" and not indicated by "a" is a portion permeated with the penetrant. The portion indicated by "A" is a portion not permeated with the iron oxide coloring liquid. The thickness of the portion indicated by "D" may be increased by dropping the penetrant. More details are described with reference to FIG. 11 and FIG. 12.

FIG. 11 shows a schematic view showing a state in which a penetrant is further dropped on a portion permeated with the penetrant in the temporary calcined body shown in FIG. 10.

FIG. 12 shows a schematic view showing a state in which a temporary calcined body is permeated with the penetrant dropped in Fig. 11. The portions indicated by "D" are portions permeated with a penetrant. The portion surrounded by the black circle and indicated by "B" and "a" is a portion permeated with the coloring liquid. The portion indicated by "B" and not indicated by "a" is a portion permeated with the penetrant. The portion indicated by "A" is a portion permeated with no coloring liquid. The number of the portion indicated by "D" is two. Therefore, the total thickness of the portions indicated by "D" is increased. Thus, the colored portion may be further moved by infiltrating various liquids, a coloring material is freely moved to a desired position.

By combining these methods, a plurality of coloring liquids which have different colors each other and a penetrant are dropped in order, therefore, an inside of a temporary calcined body may be designed freely.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this disclosure without difficulty. Accordingly, any such changes and modifications are intended to be included in the scope of the embodiments.

### Industrial Applicability

A dental crown having an inside colored in detail may be prepared by using a dental temporary calcined body and a dental calcined body of the present disclosure. Therefore, more aesthetic dental restoration than conventional one may be obtained.

### Embodiments

The present disclosure provides the following embodiments:
Embodiment 1. A dental temporary calcined body including an inside colored by a coloring material, wherein the inside of the dental temporary calcined body is colored by a coloring liquid containing the coloring material and a penetrant containing a permeating liquid.
Embodiment 2. A dental temporary calcined body including an inside colored by a coloring material, wherein the inside of the dental temporary calcined body is permeated with the coloring material by infiltrating a first coloring liquid containing a first coloring material into the dental temporary calcined body, and by infiltrating a second coloring liquid containing a second coloring material having a color different from the color of the first coloring material or a penetrant containing a permeating liquid into the dental temporary calcined body.
Embodiment 3. A method for preparing a dental temporary calcined body including an inside colored by a coloring material, wherein the method includes infiltrating a first coloring liquid containing a first coloring material into a temporary calcined body, and infiltrating a second coloring liquid containing a second coloring material having a color different from the color of the first coloring material or a penetrant containing a permeating liquid into the temporary calcined body to infiltrating the coloring material into the inside of the temporary calcined body.
Embodiment 4. A dental calcined body obtained by main calcining the dental temporary calcined body according to embodiment 1.
Embodiment 5. A dental calcined body obtained by main calcining the dental temporary calcined body according to embodiment 2.
Embodiment 6. A dental calcined body obtained by main calcining the dental temporary calcined body prepared by the method for preparing a dental temporary calcined body according to embodiment 3.
Embodiment 7. A method for preparing a dental calcined body including, main calcining the dental temporary calcined body prepared by the method for preparing a dental temporary calcined body according to embodiment 3.

### BRIEF DESCRIPTION OF THE DRAWINGS

FIG. 1 is a schematic view showing a state in which an iron oxide coloring liquid is dropped on a temporary calcined body.
FIG. 2 is a schematic view showing a state in which the temporary calcined body is permeated with the iron oxide coloring liquid.
FIG. 3 is a schematic view showing a state in which a cobalt oxide coloring liquid is dropped on a portion permeated with the iron oxide coloring liquid.
FIG. 4 is a schematic view showing a state in which the temporary calcined body is permeated with the cobalt oxide coloring liquid.
FIG. 5 is a schematic view showing a state in which a penetrant is further dropped on a portion permeated with the cobalt oxide coloring liquid.
FIG. 6 is a schematic view showing a state in which the temporary calcined body is permeated with the penetrant.
Fig. 7 is a schematic view showing a state in which an iron oxide coloring liquids are dropped on a part of the temporary calcined body.
FIG. 8 is a schematic view showing a state in which a part of the temporary calcined body is permeated with the iron oxide coloring liquid.
FIG. 9 is a schematic view showing a state in which a penetrant is dropped on a portion permeated with the iron oxide coloring liquid.
FIG. 10 shows a schematic view showing a state in which the temporary calcined body is permeated with the penetrant.
FIG. 11 is a schematic view showing a state in which a penetrant is further dropped on a portion permeated with the penetrant in the temporary calcined body.
FIG. 12 is a schematic view showing a state in which a temporary calcined body is further permeated with the penetrant.

## Claims

1. A method for preparing a dental ceramic temporary calcined body including an inside colored by a coloring material, wherein the method includes
infiltrating a first coloring liquid containing a first coloring material into the dental ceramic temporary calcined body, and
infiltrating a penetrant containing a permeating liquid into the dental ceramic temporary calcined body to infiltrate the coloring material into the inside of the dental ceramic temporary calcined body, wherein
the penetrant does not apply a coloration to the dental ceramic temporary calcined body.

2. The method for preparing a dental ceramic temporary calcined body according to claim 1, wherein
the dental ceramic temporary calcined body permeated with the first coloring liquid containing a first coloring material is temporary calcined one more times.

3. The method for preparing a dental ceramic temporary calcined body according to claim 1 or 2, wherein
the dental temporary calcined body has a block shape having a dimension of 0.5 to 3.0 cm of one side.

4. The method for preparing a dental ceramic temporary calcined body according to any one of claims 1 to 3, wherein
the dental temporary calcined body includes silica, alumina and/or zirconia in a range of 70 to 100%.

5. The method for preparing a dental ceramic temporary calcined body according to any one of claims 1 to 4, wherein
the coloring material is one or more selected from the group consisting of group 3 to 12 transition metals of the periodic table, metal salts thereof, rare earth metals and rare earth metal salts.

6. The method for preparing a dental ceramic temporary calcined body according to any one of claims 1 to 5, wherein
the coloring material is one or more selected from the group consisting of metal salts of oxides and metal salts in the form of oxides, wherein preferably
the coloring material is a metal oxide containing one or more anions selected from the group consisting of Cl⁻, SO4²⁻, SO3²⁻, Br⁻, F⁻, NO²⁻, and NO³⁻.

7. The method for preparing a dental ceramic temporary calcined body according to any one of claims 1 to 6, wherein
the coloring material is a compound including one or more selected from the group consisting of iron, manganese, copper, chrome, zirconium, cobalt, tin, titanium, nickel, vanadium, erbium, praseodymium, terbium, dysprosium, europium, niobium, neodymium and ytterbium.

8. The method for preparing a dental ceramic temporary calcined body according to any one of claims 1 to 7, wherein
a particle diameter of the coloring material is smaller than that of ceramics, and/or
a particle diameter of the coloring material is within a range of 1/4 to 1/10000 of a particle diameter of ceramics, and/or
a particle diameter of the coloring material is within a range of 0.0001 to 10 µm.

9. The method for preparing a dental ceramic temporary calcined body according to any one of claims 1 to 8, wherein
a penetrant is applied on a portion where a permeation of a coloring liquid is not desired, and permeates to prevent an invasion of a coloring liquid into a portion where a coloring is not desired, and/or
a gradation is formed by mixing the coloring liquid and the penetrant.

10. The method for preparing a dental ceramic temporary calcined body according to any one of claims 1 to 9, wherein
a penetrant is further permeated on a portion permeated with the penetrant in the temporary calcined body, and/or
a coloring liquid is further permeated on a portion permeated with the coloring liquid in the temporary calcined body, and/or
a coloring liquid is further permeated on a portion permeated with the penetrant in the temporary calcined body.

11. The method for preparing a dental ceramic temporary calcined body according to any one of claims 1 to 10, wherein
the coloring liquid contains an ionized coloring material.

12. The method for preparing a dental ceramic temporary calcined body according to any one of claims 1 to 11, wherein
the coloring liquid is a mixture containing a coloring material and a liquid material for coloring, and
the compounding ratio of a coloring material in the coloring liquid is within a range of 0.0001 to 30%, and the compounding ratio of a liquid material for coloring in the coloring liquid is within a range of 70 to 99.9999%.

13. A dental ceramic temporary calcined body prepared by the method for preparing a dental ceramic temporary calcined body according to any one of claims 1 to 12.

14. A method for preparing a dental calcined body including,
main calcining the dental temporary calcined body prepared by the method for preparing a dental ceramic temporary calcined body according to any one of claims 1 to 12.

15. A dental ceramic calcined body prepared by main calcining the dental temporary calcined body prepared by the method for preparing a dental ceramic temporary calcined body according to any one of claims 1 to 12.
